# EUROPÄISCHE PATENTSCHRIFT

(11) **EP 1 716 093 B1**
(45) Veröffentlichungstag und Bekanntmachung des Hinweises auf die Patenterteilung: **20.03.2013**
(21) Anmeldenummer: 05701101.7
(22) Anmeldetag: 21.01.2005
(51) Int. Cl.: C07C 51/08, C07C 51/06, C07C 51/377

(54) **VERFAHREN ZUR HERSTELLUNG VON ALPHA-HYDROXYCARBONS UREN UND DEREN ESTER**
METHOD FOR PRODUCING ALPHA-HYDROXYCARBOXYLIC ACIDS AND THE ESTERS THEREOF
PROCEDE POUR PREPARER DES ACIDES ALPHA-HYDROCARBOXYLIQUES ET LEURS ESTERS

(30) Priorität: 11.02.2004 DE 102004006826
(43) Veröffentlichungstag der Anmeldung: 02.11.2006
(73) Patentinhaber: Evonik Röhm GmbH, 64293 Darmstadt (DE)
(72) Erfinder: KRILL, Steffen, 67346 Speyer (DE); SCHULTHEIS, Friedel, 63594 Hasselroth (DE); GROPP, Udo, 64646 Heppenheim (DE); GROEMPING, Matthias, Kenner, CA 70065 (US)
(86) Internationale Anmeldenummer: PCT/EP2005/000578
(87) Internationale Veröffentlichungsnummer: WO 2005/077878

(56) Entgegenhaltungen:
- EP-A- 0 487 853
- DE-A1- 1 768 253
- US-A- 2 229 897
- DATABASE WPI Section Ch, Week 198238 Derwent Publications Ltd., London, GB; Class E17, AN 1982-80265E XP002329272 & JP 57 131736 A (MITSUBISHI GAS CHEM IND CO LTD) 14. August 1982 (1982-08-14) in der Anmeldung erwähnt

## Beschreibung

Die vorliegende Erfindung betrifft ein Verfahren zur selektiven, wirtschaftlichen Herstellung von alpha-Hydroxycarbonsäuren und deren Ester und hiervon abgeleitete Dehydratisierungsprodukte, insbesondere Methacrylsäure und Methacrylsäureestern, ausgehend von Cyanhydrinen.

Methacrylsäure und Methacrylsäureester finden ihr Haupteinsatzgebiet in der Herstellung von Polymeren und Copolymeren mit anderen polymerisierbaren Verbindungen. Methacrylsäureester, wie zum Beispiel Methylmethacrylat, ist zudem ein wichtiger Baustein für diverse, auf Methacrylsäure basierender Spezialester, die durch Umesterung mit dem entsprechenden Alkohol hergestellt werden.

Methylmethacrylat (MMA) und Methacrylsäure werden heute überwiegend ausgehend von Blausäure und Aceton über das entstehende Acetoncyanhydrin (ACH) als Zentralintermediat hergestellt.

Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute C-4 basierte Rohstoffe wie Isobutylen oder tert-Butanol als Edukte verwendet, die über mehrere Verfahrenstufen in die gewünschten Methacrylsäurederivate umgewandelt werden.

Intensiv untersucht wurde darüber hinaus die Verwendung von Propen als Basisrohstoff, wobei man über die Stufen Hydrocarbonylierung (zur Isobuttersäure) und dehydrierender Oxidation in moderaten Ausbeuten zur Methacrylsäure gelangt.

Es ist bekannt, Propanal oder Propionsäure, die in technischen Prozessen ausgehend von Ethylen und C-1 Bausteinen wie Kohlenmonoxid zugänglich sind, als Basisrohstoff einzusetzen. In diesen Prozessen wird in einer aldolisierenden Reaktion mit Formaldehyd unter Dehydratisierung der in situ entstehenden β-Hydroxycarbonylverbindung zur entsprechenden α, ß-ungesättigten Verbindung umgesetzt. Eine Übersicht über die gängigen Verfahren zur Herstellung der Methacrylsäure und deren Ester findet sich in der Literatur wie Weissermel, Arpe "Industrielle organische Chemie", VCH, Weinheim 1994, 4. Auflage, S.305 ff oder Kirk Othmer "Encyclopedia of Chemical Technology", 3. Ausgabe, Vol. 15, Seite 357.

Es ist allgemein bekannt, dass technische, auf ACH basierende Verfahren mit hochkonzentrierter Schwefelsäure (um etwa 100 Gew.-% H₂SO₄) im ersten Schritt der Umsetzung, der sogenannten Amidierung, bei Temperaturen zwischen 80°C bis etwa 110°C durchgeführt werden.

Repräsentativ für einen solchen Prozess ist beispielsweise US Patent 4,529,816, in der die ACH Amidierung bei Temperaturen um 100°C mit einem molaren Verhältnis von ACH : H₂SO₄ von etwa 1 1,5 bis 1 : 1,8 durchgeführt wird. Verfahrensrelevanten Prozessschritte für diesen Prozess sind: a) Amidierung; b) Konvertierung; und c) Veresterung.

In der Amidierung werden als Hauptprodukte der Umsetzung SIBA = Sulfoxy-alpha-Hydroxyisobuttersäureamid-Hydrogensulfat und MASA x H₂SO₄ = Methacrylsäureamid-Hydrogensulfat als Lösung in überschüssiger Schwefelsäure erhalten. Des Weiteren ist in einer typischen Amidierungslösung noch HIBA x H₂SO₄ = alpha-Hydroxyisobuttersäureamid-Hydrogensulfat mit einer Ausbeute bezüglich ACH von < 5% erhalten. Bei mehr oder weniger vollständigem ACH Umsatz verläuft dieser an sich recht selektive Amidierungsprozess mit einer Ausbeute (= Summe der beschriebenen Intermediate) von ca. 96-97%.

Als Nebenprodukte in diesem Schritt werden damit aber bereits in nicht unerheblichen Mengen Kohlenmonoxid, Aceton, Sulfonierungsprodukte des Acetons und Cyclokondensationsprodukte von Aceton mit diversen Intermediaten gebildet.

Ziel der Konvertierung ist die möglichst vollständige Umsetzung von SIBA und HIBA zu MASA, die unter β-Eliminierung von Schwefelsäure (in überschüssiger Schwefelsäure als Lösungsmittel) verläuft.

In dem Verfahrensschritt Konvertierung wird nun die schwefelsaure (wasserfreie) Lösung aus HIBA, SIBA und MASA (liegen jeweils als Hydrogensulfate vor) in der sogenannten Konvertierung bei hohen Temperaturen zwischen 140°C - 160°C und kurzen Verweilzeiten von etwa 10 min oder weniger umgesetzt.

Das Konvertierungsgemisch dieser Verfahrensweise ist charakterisiert durch einen hohen Überschuss Schwefelsäure und das Vorliegen des Hauptprodukts MASA x H₂SO₄ mit einer Konzentration in der Lösung von etwa 30-35 Gew.-% (je nach eingesetztem Schwefelsäureüberschuss).

Je nach Wassergehalt in der verwendeten Schwefelsäure stellt sich auch der Anteil von HIBA neben SIBA in der Amidierungsmischung ein. Verwendet man beispielsweise eine 97 Gew.-%ige Schwefelsäure (1,5 Äquivalente H₂SO₄ bzgl. ACH), so entstehen bereits um die 25 Gew.-% HIBA, die nicht mehr selektiv und vollständig in der Konvertierung zu MASA umgesetzt werden können. Durch den relativ hohen Wasseranteil in der Amidierung bei Temperaturen von 90°C - 110°C bedingt sich also ein relativ hoher Anteil an HIBA, der durch konventionelle Konvertierung nur relativ unselektiv zum Zielintermediat MASA x H₂SO₄ umgewandelt werden kann.

Bei mehr oder weniger vollständigem SIBA x H₂SO₄ Umsatz verläuft der Konvertierungsschritt mit einer MASA x H₂SO₄ Ausbeute von ca. 94-95%. Zuzüglich der Verluste in der Amidierung durch die oben beschriebenen Nebenreaktionen stehen damit für die anschließende Veresterung zum als Produkt gewünschten Methylmethacrylat (MMA) nur zwischen 90-92% MASA (bzgl. ACH) zur Verfügung.

Als Nebenprodukte in diesem Verfahrensschritt werden bedingt durch die drastischen Reaktionsbedingungen erhebliche Mengen an Kondensations- und Additionsprodukten der Intermediate miteinander gebildet.

Ziel der Veresterung ist die möglichst vollständige Umsetzung von MASA x H₂SO₄ aus der Konvertierung zu MMA. Die Veresterung verläuft durch Zugabe eines Gemisches bestehend aus Wasser und Methanol zur MASA - Schwefelsäure Lösung und verläuft zumindest teilweise über Methacrylsäure (MAS) als Intermediat. Die Reaktion kann unter Druck oder drucklos betrieben werden.

Üblicherweise wird hierbei durch Verseifung/Veresterung der Konvertierungslösung bei Temperaturen zwischen 90°C - 140°C bei Reaktionszeiten von ein oder mehreren Stunden eine schwefelsaure Lösung von MMA, MAS und gebildetem Ammoniumhydrogensulfat erhalten.

Durch die Reaktionsbedingungen in Anwesenheit freier Schwefelsäure beträgt die Methanolselektivität in diesem Schritt nur etwa 90% oder weniger, wobei Dimethylether durch Kondensation von Methanol als Nebenprodukt gebildet wird.

Bei mehr oder weniger vollständigem MASA x H₂SO₄ Umsatz verläuft die Veresterung mit einer MMA Ausbeute von ca. 98-99% bezüglich eingesetztem MASA (summarische Selektivität von MAS + MMA). Zuzüglich der Verluste in der Amidierung und der Konvertierung durch die oben beschriebenen Nebenreaktionen können damit im Gesamtprozess über alle Stufen MMA Ausbeuten von maximal 90% bezüglich ACH bei optimaler Reaktionsführung erreicht werden.

Neben den schlechten Gesamtausbeuten des oben beschriebenen Prozesses, die insbesondere im Produktionsmaßstab mit dem Anfall erheblicher Mengen an Abfällen und Abgasen verbunden ist, hat dieser Prozess den Nachteil, dass weit überstöchiometrische Mengen an Schwefelsäure eingesetzt werden müssen. Aus der Ammoniumhydrogen- und Schwefelsäure haltigen Prozesssäure, die in der Schwefelsäurekontaktanlage regeneriert wird, sondern sich zudem teerartige, feste Kondensationsprodukte ab, die eine einwandfreie Förderung der Prozesssäure behindern und unter erheblichem Aufwand beseitigt werden müssen.

Aufgrund der drastischen Ausbeuteverluste bei dem oben beschriebenen Verfahren aus US Patent 4,529,816, gibt es einige Vorschläge, ACH in Gegenwart von Wasser zu amidieren und hydrolysieren, wobei die Hydroxyfunktion im Molekülverbund zumindest in den ersten Schritten der Umsetzung erhalten bleibt.

Diese Vorschläge zu einer alternativen Amidierung in Gegenwart von Wasser führen je nach dem, ob in Gegenwart von oder ohne Methanol durchgeführt, entweder zur Entstehung von Hydroxyisobuttersäuremethylester (= HIBSM) oder zur Entstehung von 2-Hydroxyisobuttersäure (= HIBS).

Hydroxyisobuttersäure ist ein zentrales Intermediat für die Herstellung von Methacrylsäure und hiervon abgeleitete Methacrylsäureester, insbesondere Methylmethacrylat, die aufgrund ihrer Anwendung als Monomerbausteine für die Produktion diverser Kunststoffe große technische Bedeutung gewonnen haben.

Ein weitere Alternative der Herstellung von Estern der alpha-Hydroxyisobuttersäure, insbesondere alpha-Hydroxyisobuttersäuremethylester, ausgehend von ACH wird in JP Hei- 4-193845 beschrieben. In JP Hei- 4-193845 wird ACH mit 0,8 bis 1,25 Äquivalenten Schwefelsäure in Gegenwart von weniger als 0,8 Äquivalenten Wasser unterhalb 60°C zunächst amidiert und anschließend bei Temperaturen von größer als (>) 55°C mit mehr als 1,2 Äquivalenten Alkohol, insbesondere Methanol, zum HIBSM oder entsprechenden Estern umgesetzt. Auf die Anwesenheit von viskositätserniedrigenden Medien, die stabil gegenüber der Reaktionsmatrix sind, wird hier nicht eingegangen.

Die Nachteile und Probleme dieses Verfahrens sind die technische Umsetzung durch außerordentliche Viskositätsbildung am Ende der Reaktion.

Einige Ansätze zur Verwertung und Umwandlung von HIBSM (=alpha-Hydroxyisobuttersäuremethylester) durch Dehydratisierung zu Methylmethacrylat sind in der Patentliteratur beschrieben.

Zum Beispiel, in der EP 0 429 800 wird HIBSM oder eine Mischung aus HIBSM und eines entsprechenden alpha- oder beta-Alkoxyesters in der Gasphase, in Gegenwart von Methanol als Co-Feed an einem heterogenen Katalysator bestehend aus einem kristallinen Alumosilikat und einer Misch-Dotierung aus einem Alkalimetallelement einerseits und einem Edelmetall andererseits umgesetzt. Obwohl Umsatz und Selektivität des Katalysators zumindest zu Beginn der Reaktion ganz gut sind, kommt es mit zunehmender Reaktionszeit zu einer recht drastischen Deaktivierung des Katalysators, die mit absinkenden Ausbeuten einhergeht.

Einen ähnlichen Ansatz verfolgt EP 0 941 984, in der die Gasphasen-Dehydrierung von HIBSM als Teilschritt einer MMA Synthese in Gegenwart eines heterogenen Katalysators bestehend aus einem Alkalimetallsalz der Phosphorsäure auf SiO₂ beschrieben wird. Insgesamt ist aber dieses vielstufige Verfahren kompliziert, erfordert in Teilschritten erhöhte Drücke und damit teures Equipment und liefert nur unbefriedigende Ausbeuten.

Neben den oben dargestellten Arbeiten zur Dehydratisierung von HIBSM und verwandter Ester zu den entsprechenden alphabeta ungesättigten Methacrylsäureverbindungen in der Gasphase, gibt es auch Vorschläge zur Durchführung der Reaktion in flüssiger Phase, wie beispielsweise in US 3,487,101.

In JP 184047/1985 wird weiterhin die Dehydratisierung von HIBSM in Gegenwart hochkonzentrierter Schwefelsäure (90 - 100 Gew.- %). Nachteilig hierbei sind die hohen Aufwandmengen an Schwefelsäure und der Zwangsanfall großer Mengen wässriger Schwefelsäure, die im Verlauf der Reaktion durch die Wasserfreisetzung aus HIBSM gebildet wird. Eine wirtschaftliche Bedeutung erlangt dieses Verfahren aufgrund der Abfallsäuremengen nicht.

Die Herstellung von MAS ausgehend von Hydroxyisobuttersäure wird, zum Beispiel in US 3,487,101 beschrieben, wo die Herstellung diverser Methacrylsäurederivate, insbesondere Methacrylsäure und Methacrylsäureester, ausgehend von Hydroxyisobuttersäure in der Flüssigphase, dadurch gekennzeichnet ist, dass die Umsetzung von HIBS zu Methacrylsäure in Gegenwart eines gelösten basischen Katalysators bei hohen Temperaturen zwischen 180°C - 320°C in Gegenwart hochsiedender Ester (z.B. Phtalsäuredimethylester) und inneren Anhydriden (z.B. Phtalsäureanhydrid) durchgeführt wird. Laut Patent werden bei HIBS Umsätzen > 90% MAS-Selektivitäten um 98% erreicht. Über die Langzeitstabilität der flüssigen Katalysatorlösung insbesondere der Erschöpfung des eingesetzten Anhydrids werden keine Angaben gemacht.

DE-OS 1 191367 betrifft die Herstellung von Methacrylsäure ausgehend von Hydroxyisobuttersäure in der Flüssigphase, dadurch gekennzeichnet, dass die Umsetzung von HIBS zu Methacrylsäure in Gegenwart von Polymerisationsinhibitoren (wie z.B. Kupferpulver) in Gegenwart eines Katalysatorgemischs bestehend aus Metallhalogeniden und Alkalihalogeniden bei hohen Temperaturen zwischen 180 - 220°C durchgeführt wird. Laut Patent werden bei HIBS Umsätzen > 90% MAS-Selektivitäten von > 99% erreicht. Die besten Ergebnisse werden mit Katalysatormischungen aus Zinkbromid und Lithiumbromid erreicht. Es ist allgemein bekannt, dass die Verwendung halogenid-haltiger Katalysatoren bei hohen Temperaturen drastische Forderungen an die zu verwendenden Werkstoffe stellt und diese Probleme bezüglich der im Destillat befindlichen, halogenierten verschleppten Nebenprodukte auch in nachfolgenden Anlagenteilen auftreten.

EP 0 487 853 beschreibt die Herstellung von Methacrylsäure ausgehend von Acetoncyanhydrin, dadurch gekennzeichnet, dass man im ersten Schritt ACH mit Wasser bei moderaten Temperaturen in Gegenwart eines heterogenen Hydrolysekatalysators umsetzt und man im zweiten Schritt Hydroxyisobuttersäureamid mit Methylformiat oder Methanol/Kohlenmonoxid unter Entstehung von Formamid und Hydroxyisobuttersäuremethylester umsetzt, und man im dritten Schritt HIBSM in Gegenwart eines heterogenen Ionenaustauschers mit Wasser zu Hydroxyisobuttersäure verseift, und man im vierten Schritt HIBS dehydratisiert, indem man in flüssiger Phase bei hohen Temperaturen in Gegenwart eines löslichen Alkalisalzes reagieren lässt. Die Methacrylsäure-Herstellung ex HIBS wird bei hohen Umsätzen um 99% mit mehr oder weniger quantitativen Selektivitäten beschrieben. Die Vielzahl der notwendigen Reaktionsschritte und die Notwendigkeit der Zwischenisolierung einzelner Intermediate, insbesondere auch die Durchführung einzelner Prozessschritte bei erhöhtem Druck, machen das Verfahren kompliziert und damit letztlich unwirtschaftlich.

DE-OS 1 768 253 beschreibt ein Verfahren zur Herstellung von Methacrylsäure durch Dehydratisierung von alpha-Hydroxyisobuttersäure, dadurch gekennzeichnet, dass man HIBS in flüssiger Phase bei einer Temperatur von wenigstens 160°C in Gegenwart eines Dehydratisierungskatalysators umsetzt, der aus einem Metallsalz von alpha-Hydroxyisobuttersäure besteht. Besonders geeignet sind in diesem Fall die Alkali- und Erdalkalisalze von HIBS, die in einer HIBS-Schmelze durch Umsetzung geeigneter Metallsalze in situ hergestellt werden. Laut Patent werden MAS Ausbeuten bis 95% ex HIBS beschrieben, wobei der Feed der kontinuierlichen Verfahrensweise aus HIBS und ca. 1,5 Gew.-% HIBS-Alkalisalz besteht.

RU 89631 betrifft ein Verfahren zur Herstellung von Methacrylsäure ausgehend von Hydroxyisobuttersäure durch Wasserabspaltung in flüssiger Phase, dadurch gekennzeichnet, dass die Reaktion in Abwesenheit eines Katalysators mit einer wässrigen Lösung von HIBS (bis 62 Gew.-% HIBS in Wasser) unter Druck bei hohen Temperaturen 200°C - 240°C durchgeführt wird.

Es ist außerdem bekannt, dass zur Herstellung von Hydroxyisobuttersäure ausgehend von Acetoncyanhydrin (ACH) die Verseifung der Nitrilfunktion in Gegenwart von Mineralsäuren durchgeführt werden kann (siehe US 222989; J. Brit. Chem. Soc. (1930); Chem. Ber. 72 (1939), 800].

Repräsentativ für einen solchen Prozess ist beispielsweise die japanische Patentveröffentlichung Sho 63-61932, in der ACH in einem zweistufigen Prozess zur Hydroxyisobuttersäure verseift wird. Hierbei wird ACH zunächst in Gegenwart von 0,2 -1,0 mol Wasser und 0,5 - 2 Äquivalenten Schwefelsäure umgesetzt, wobei die entsprechenden Amidsalze gebildet werden. Bereits in diesem Schritt treten bei Verwendung geringer Wasser- und Schwefelsäurekonzentrationen, die zum Erhalt guter Ausbeuten, kurzer Reaktionszeiten und geringer Abfall-Prozesssäuremengen notwendig sind, massive Probleme mit der Rührbarkeit des Amidierungsgemischs durch hohe Viskosität der Reaktionsansätze insbesondere gegen Ende der Reaktionszeit auf.

Erhöht man die molare Wassermenge zur Gewährleistung einer niedrigen Viskosität, so verlangsamt sich die Reaktion drastisch und es treten Nebenreaktionen auf, insbesondere die Fragmentierung von ACH in die Edukte Aceton und Blausäure, die unter den Reaktionsbedingungen zu Folgeprodukten weiterreagieren. Auch bei Erhöhung der Temperatur lässt sich nach den Vorgaben der japanischen Patentveröffentlichung SHO 63-61932 die Viskosität des Reaktionsgemischs zwar beherrschen und die entsprechenden Reaktionsansätze werden durch die sinkende Viskosität zwar rührbar, aber auch hier nehmen bereits bei moderaten Temperaturen die Nebenreaktionen drastisch zu, was sich letztlich in nur mäßigen Ausbeuten äußert (siehe Vergleichsbeispiele).

Arbeitet man bei niedrigen Temperaturen < 50°C, die eine selektive Reaktionsführung gewährleisten würde, so kommt es gegen Ende der Reaktionszeit durch die Erhöhung der Konzentration der unter den Reaktionsbedingungen schwer löslichen Amidsalzen zunächst zur Bildung einer schwer rührbaren Suspension und schließlich zur vollständigen Verfestigung des Reaktionsansatzes.

Im zweiten Schritt der japanischen Patentveröffentlichung SHO 63-61932 wird Wasser zur Amidierungslösung gegeben und bei höheren Temperaturen als der Amidierungstemperatur hydrolisiert, wobei sich aus den nach der Amidierung gebildeten Amidsalzen unter Freisetzung von Ammoniumhydrogensulfat Hydroxyisobuttersäure bildet.

Wesentlich für die Wirtschaftlichkeit eines technischen Prozesses ist neben der selektiven Darstellung des Zielprodukts HIBS in der Reaktion auch die Isolierung aus der Reaktionsmatrix bzw. die Abtrennung von HIBS von der verbleibenden Prozesssäure.

In JP Sho 57-131736, Methode zur Isolierung von alpha-Oxyisobuttersäure (=HIBS), wird diese Problematik behandelt, indem die nach der Reaktion zwischen Acetoncyanhydrin, Schwefelsäure und Wasser durch hydrolytische Spaltung erhaltene, alpha-Hydroxyisobuttersäure und saures Ammoniumhydrogensulfat enthaltende Reaktionslösung mit einem Extraktionsmittel behandelt wird, wobei die Hydroxyisobuttersäure in das Extraktionsmittel übergeht und das saure Ammonsulfat in der wässrigen Phase zurückbleibt.

Nach diesem Verfahren, wird vor der Extraktion die noch freie Schwefelsäure im Reaktionsmedium durch Behandlung mit einem alkalischen Medium neutralisiert, um den Extraktionsgrad von HIBS in die organische Extraktionsphase zu erhöhen. Die notwendige Neutralisation ist mit einem erheblichen Mehraufwand an aminischer oder mineralischer Base verbunden und damit mit erheblichen Abfallmengen an entsprechenden Salzen, die nicht ökologisch und wirtschaftlich entsorgt werden können.

Die Nachteile des JP Sho 57-131736 Verfahrens zur Darstellung von MMA über Methacrylsäureamid-Hydrogensulfat (Reaktionssequenz: Amidierung - Konvertierung - hydrolytische Veresterung) lassen sich folgendermaßen zusammenfassen:
a.) Verwendung hoher molarer Schwefelsäure Überschüsse bezüglich ACH (im technischen Prozess ca. 1,5 - 2 Äquivalente Schwefelsäure pro Äquivalent ACH)
b.) Hohe Ausbeuteverluste im Amidierungsschritt (ca. 3-4%) und im Konvertierungsschritt (ca. 5-6%), was sich letztlich in einer maximalen Methacrylsäureamidsulfat Ausbeute von ca. 91% äußert.
c.) Große Abfallströme in Form wässriger Schwefelsäure, in der Ammoniumhydrogensulfat und organische Nebenprodukte gelöst sind. Abscheidung undefinierter Teerrückstände aus dieser Prozess-Abfallssäure, die eine Nachbehandlung bzw. aufwendige Entsorgung notwendig machen.

Die Nachteile des JP Sho 57-131736 Verfahrens zur Darstellung von MMA über Hydroxyisobuttersäure als Zentralintermediat (Reaktionssequenz: Amidierung - Hydrolyse; HIBS Synthese - MAS Synthese- hydrolytische Veresterung) lassen sich folgendermaßen zusammenfassen:
a.) Verwendung zwar geringer molarer Schwefelsäure Überschüsse bezüglich ACH (nur ca. 1,0 Äquivalente Schwefelsäure pro Äquivalent ACH), aber massive Probleme mit Viskosität und Rührbarkeit des Amidierungsmediums bis zur kompletten Verfestigung der Reaktionsansätze; die vorgeschlagene Verdünnung der Amidierung mit Alkoholen (Methanol) oder diversen Estern führt unter den Reaktionsbedingungen zur unvollständigen ACH Umsetzung, drastischer Zunahme der Nebenreaktionen oder zur chemischen Zersetzung der Verdünner;
b.) Hohe Ausbeuteverluste im Amidierungsschritt (ca. 5-6%) und aufwendige Extraktion mit einem organischen Lösungsmittel unter Entstehung einer Wasser - und HIBS enthaltenden Extraktionsmittelphase, die unter hohem energetischen Aufwand zur Isolierung von HIBS destillativ aufbereitet werden muss. Pro kg HIBS werden etwa 2 kg Prozesssäure-Abfall erzeugt, der etwa 34 Gew.-% Wasser neben 66 Gew.-% Ammoniumhydrogensulfat enthält (siehe japanische Offenlegung SHO-57-131736, Beispiel 4). Die Regenerierung einer Abfallsalzlösung mit hohen Wassergehalten in einer Schwefelsäure-Kontaktanlage (=SK Anlage) ist mit einem erheblichen energetischen Aufwand verbunden, die Kapazität einer solchen SK Anlage deutlich limitiert.

Allen diesen Verfahren ist gemeinsam, dass die Isolierung von HIBS aus der Ammoniumhydrogensulfat-haltigen wässrigen Reaktionsmatrix sehr aufwendig ist. Ein zu hoher Wassergehalt in der HIBS-haltigen Extraktionsphase bedingt auch eine Verschleppung von Ammoniumhydrogensulfat in die nachfolgende MAS Stufe, die nicht mehr im technischen Maßstab über einen vertretbaren Zeitraum hinweg kontinuierlich betrieben werden kann. Der hohe energetische Aufwand bei der Regenerierung hochkonzentrierter wasserhaltiger Prozesssäure- wie auch Extraktionsströme machen die vorgeschlagenen Verfahrensweisen zudem unwirtschaftlich und bieten gegenüber dem zwar unselektiven, aber aufgrund der einfachen, wenigen verfahrenstechnischen Operationen zielgerichteten, etablierten Verfahrensweise, keine wirkliche Alternative.

Damit war es Aufgabe dieser Erfindung, ein Verfahren zur Herstellung von Methacrylsäure und entsprechenden Estern ausgehend von Cyanhydrin zu finden, bei dem
a.) eine für die technische Durchführung geeignete Viskosität im Amidierungsgemisch gewährleistet ist durch Wahl eines geeigneten inerten, leicht abtrennbaren Lösungsmittels bei gleichzeitiger Sicherstellung einer schnellen, hochselektiven Produktbildung,
b.) hohe Ausbeuten an Hydroxyisobuttersäure und MAS ausgehend von ACH (> 95%) erreicht werden,
c.) Reaktionszeiten unter 60 min zur Darstellung von Amidsulfat-Intermediaten (=Amidierung) und unter 120 min für die Hydrolyse (= HIBS Herstellung ex Amidsulfaten) erzielt werden,
d.) eine Reduzierung der Prozesssäure Abfallmengen durch Umsetzung von ACH mit mehr oder weniger stöchiometrischen Schwefelsäuremengen erreicht wird, und
e.) eine Reduzierung und Vereinfachung der notwendigen verfahrenstechnischen Operationen durch Verschaltung der Reaktionsstufe von HIBS und MAS erzielt wird.

Eine weitere Aufgabe dieser Erfindung war es, ausgehend von Acetoncyanhydrin, Hydroxyisobuttersäure (HIBS) und Methacrylsäure (MAS) in hoher Selektivität und Ausbeute mit minimalem Verbrauch der in der Amidierungsstufe verbrauchten Schwefelsäure herzustellen und eine einfache Methode zur Isolierung sowohl von HIBS als auch von MAS ohne aufwendige verfahrenstechnische Operationen ohne Zugabe weiterer Hilfsstoffe zu gewährleisten.

Ein Aspekt dieser Erfindung ist ein Verfahren zur selektiven, wirtschaftlichen Herstellung von Methacrylsäure und Methacrylsäureestern ausgehend von Acetoncyanhydrin (ACH), wobei in einem ersten Schritt durch Umsetzung von ACH mit Schwefelsäure in Gegenwart von Wasser und einem geeigneten polaren Lösungsmittel 2-Hydroxy-2-methylpropionsäure (2-Hydroxyisobuttersäure) hergestellt und isoliert wird und anschließend durch ß-Eliminierung von Wasser ausgehend von 2-Hydroxy-2-methylpropionsäure Methacrylsäure hergestellt wird. In einem dritten, optionalen, Schritt kann die so erhaltene Methacrylsäure nach an sich bekannten Verfahren mit diversen Alkoholen zu den entsprechenden Methacrylsäureestern umgesetzt werden.

Ein weiterer Aspekt der vorliegende Erfindung betrifft insbesondere ein Verfahren zur hochselektiven Herstellung von Hydroxyisobuttersäure ausgehend von Acetoncyanhydrin über die Reaktionssequenz Amidierung und Hydrolyse, wobei ausgehend von Acetoncyanhydrin, 2-Hydroxyisobuttersäureamid in Form eines Salzes mit Schwefelsäure als Intermediat durchlaufen wird, das ohne Isolierung durch Hydrolyse zum gewünschten Produkt 2-Hydroxyisobuttersäure verseift wird.

Ausgehend von 2-Hydroxyisobuttersäure kann Methacrylsäure durch Wasserabspaltung (ß-Eliminierung) in flüssiger Phase in Gegenwart geeigneter Metallkatalysatoren in hoher Ausbeute und hoher Reinheit hergestellt werden.

Der vorliegende Erfindung erlaubt die hochselektive Herstellung von Methacrylsäure in bislang nicht erreichbaren Ausbeuten bezüglich ACH von > 97% bei gleichzeitiger drastischer Reduzierung der für die Umsetzung notwendigen Schwefelsäuremengen.

Die vorliegende Erfindung ist ein Verfahren zur Herstellung von Methacrylsäure, dadurch gekennzeichnet, dass man
a) Acetoncyanhydrin bei Temperaturen unter 80°C mit maximal 1,5 Äquivalenten Schwefelsäure in Gegenwart von 0,05 - 1,0 Äquivalenten Wasser in Gegenwart eines unter den Reaktionsbedingungen inerten, polaren Lösungsmittels unter Entstehung einer gut rührbaren Lösung der entsprechenden Amidsulfate im inerten, polaren Lösungsmittel umsetzt,
b) nach Zugabe von Wasser diese Lösung in Gegenwart oder nach vorheriger Entfernung des inerten, polaren Lösungsmittels zu einer Lösung bestehend im wesentlichen aus Wasser, Ammoniumhydrogensulfat und alpha-Hydroxyisobuttersäure umsetzt,
c) Hydroxyisobuttersäure durch Extraktion mit einem geeigneten Extraktionsmittel von der wässrigen Ammoniumhydrogensulfatlösung abtrennt,
d) nach Entfernung des Extraktionsmittels die in hoher Konzentration gewonnene alpha-Hydroxyisobuttersäure in Gegenwart eines Metallsalzes der alpha-Hydroxyisobuttersäure bei Temperaturen zwischen 160°C - 300°C in flüssiger Phase zu einem als Destillat anfallenden Gemisch bestehend aus im wesentlichen Methacrylsäure und Wasser umsetzt, und
e) Methacrylsäure destillativ aus diesem Gemisch in hochreiner Form gewinnt oder das unter d) anfallende Produktgemisch (MAS-Wasser) als Extraktionsmittel für die Isolierung der alpha-Hydroxyisobuttersäure nach Schritt c) einsetzt und anschließend die Wertstoffe destillativ voneinander trennt.

In einem Aspekt der Erfindung kann man zusätzlich zu oben beschriebenen Schritten a) bis e) Schritt f) durchführen:
f) das unter Schritt c) erhaltene Gemisch aus Methacrylsäure/Wasser oder reiner Methacrylsäure selbst wird mit einem Alkohol umgesetzt, wobei der als Produkt gewünschte Ester (= Methacrylsäureester) in einer für die Anwendung gewünschte Form nach bekannten Methoden gewonnen wird.

Das Verfahren kennzeichnet sich im ersten Schritt a) durch die Umsetzung von Acetoncyanhydrin mit Schwefelsäure in Gegenwart von Wasser und einem geeigneten inerten Lösungsmittel zur Darstellung von Hydroxyisobuttersäureamid und im zweiten Schritt b) durch die Verseifung des *in situ* gebildeten Amids durch Umsetzung mit Wasser.

Weiterhin zeichnet sich das erfindungsgemäße Verfahren dadurch aus, dass bei Reaktionszeiten für die Amidierung unter 60 min, vorzugsweise unter 45 min, vorzugsweise unter 30 min, besonders bevorzugt unter 20 min liegen, und bei Reaktionszeiten für die Hydrolyse unter 120 min, vorzugsweise unter 100 min, besonders bevorzugt unter 75 min, vollständige Umsätze von größer als (>) 99% erzielt werden.

Im Schritt a) werden erfindungsgemäß, Temperaturen unter 80°C, vorzugsweise zwischen 20°C und 80°C, vorzugsweise unter 70°C, besonders bevorzugt unter 60°C angewandt.

Erfindungsgemäß wird die Schwefelsäure mit maximal 1,5 Äquivalente (bezüglich ACH), vorzugsweise zwischen 0,5 und 1,5 Äquivalenten, vorzugsweise 0,8 bis 1,5 Äquivalenten und besonders bevorzugt zwischen 0,9 und 1,1 Äquivalente, eingesetzt.

Erfindungsgemäß, wird im Schritt a) Wasser in einer Menge von 0,05 bis 1,0 Äquivalente, bezüglich ACH, vorzugsweise 0,1 bis 05 Äquivalente eingesetzt.

In der Verseifungsstufe, Verfahrensschritt b), wird erfindungsgemäß Wasser in einer Menge von 0,5 bis 10 Äquivalente, bezüglich ACH, vorzugsweise 1,0 bis 6,5 Äquivalente, vorzugsweise 1,0 bis 4 Äquivalente eingesetzt.

Ein weiteres wesentliches Kennzeichen des erfindungsgemäßen Verfahrens ist die vorteilhafte Rückführung bzw. Kreislaufführung des inerten Lösungsmittels. Das erfindungsgemäße Verfahren kennzeichnet sich weiterhin durch eine hochselektive Reaktionsführung, die den Nebenproduktanfall des Verfahrens auf ein Minimum reduziert, woraus sich letztlich deutliche wirtschaftliche Vorteile ableiten.

Durch die erfindungsgemäße Arbeitsweise der beschriebenen Reaktionsschritte gelingt es in einfacher Weise Hydroxyisobuttersäure (HIBS) hochselektiv bezüglich dem als Edukt verwendeten Acetoncyanhydrin in Ausbeuten von mindestens 95%, vorzugsweise größer als 95%, bevorzugt größer als 98% und besonders bevorzugt bis zu 99,5% herzustellen und gleichzeitig die Schwefelsäuremengen im Vergleich zu den Verfahren aus dem Stand-der-Technik erheblich zu reduzieren.

Ein weiterer deutlicher Vorteil gegenüber bislang praktizierten Verfahren, die im Stand der Technik erläutert werden, sind kurze Reaktionszeiten der einzelnen Verfahrensstufen, die Raum-Zeit Ausbeuten von größer als 95% gewährleisten.

Durch die im Vergleich mit den bekannten Verfahren wesentlich geringeren Schwefelsäuremengen, wird eine nachgeschaltete Schwefelsäure-Kontakt Anlage, in der der Ammoniumsulfat-haltige wässrige Abfallstrom zur Regenerierung von Schwefelsäure behandelt wird, deutlich entlastet. Bei gegebener Kapazität der Schwefelsäurekontaktanlage hat dies eine deutliche Mehrproduktion an Wertstoff (Methacrylsäure und Methacrylsäurederivate, im wesentlichen Methylmethacrylat) zur Folge.

Die nach dem erfindungsgemäßen Verfahren entstehende Prozesssäure kann in einfacher Weise in einer Schwefelsäurekontakteinheit regeneriert und in den Prozess zurückgeführt werden.

Exemplarisch für inerte, polare Lösungsmittel, die als Viskositätsmoderator für in situ entstehende Hydroxycarbonsäureamide und Hydroxycarbonsäuren einsetzbar sind, sind inerte C₂-C₁₂ Carbonsäuren, aliphatische Sulfonsäuren und davon abgeleitete Ester oder inerte Nitroverbindungen.

Erfindungsgemäße C₂-C₁₂ Carbonsäuren sind Carbonsäuren ausgewählt aus der Gruppe von Essigsäure, Propionsäure, Methylpropansäure, Buttersäure, Isobuttersäure und entsprechende homologe höherkettige, aliphatische verzweigte und unverzweigte Carbonsäuren, wobei Essigsäure besonders bevorzugt ist. Neben den hier beschriebenen Carbonsäuren können auch die davon abgeleiteten Ester eingesetzt werden, wie beispielsweise die entsprechenden Methyl-, Ethyl-, Propyl-, Isopropyl- oder höhere Ester mit C-4 bis C-10 Kohlenstoffatomen. Exemplarisch seien hier Acetatester der hier aufgezählten Carbonsäuren genannt.

Aus der Gruppe der Ester, sind die bevorzugten Lösungsmittel oder Viskositätsmoderatoren die in der Amidierung eingesetzt werden können, die Ester der alpha-Hydroxyisobuttersäure, wobei der alpha-Hydroxyisobuttersäuremethylester besonders bevorzugt als Lösungsmittel der Amidierung eingesetzt wird, da er auch als Intermediat im Prozess entsteht und deshalb teilweise in die erste Stufe (Amidierung) zurückgeführt werden kann.

Methacrylsäure selbst oder entsprechende analoge und homologe Verbindungen können auch als inertes, polares Lösungsmittel oder Viskositätsmoderatoren aus der Gruppe der Carbonsäuren eingesetzt werden.

Erfindungsgemäße aliphatische Sulfonsäuren sind Methansulfonsäure, Ethansulfonsäure, Propansulfonsäure sowie entsprechende homologe Verbindungen mit einem Kohlenwasserstoffrest mit C-3 - C-12 Kohlenstoffatomen, die gegebenenfalls substituiert oder verzweigt sein können. Erfindungsgemäße substituierte, aliphatische Sulfonsäuren können aus der Gruppe der Halogenoalkansulfonsäuren gewählt werden, wie zum Beispiel Trifluormethansulfonsäure und höhere homologe Verbindungen davon. Neben diesen hier beschriebenen Carbonsäuren können auch die davon abgeleiteten Ester eingesetzt werden, wie beispielsweise die entsprechenden Methyl-, Ethyl-, Propyl-, Isopropyl-oder höhere Ester mit C-4 bis C-10 Kohlenstoffatomen. Exemplarisch seien hier der Methylester der hier aufgezählten Sulfonsäuren genannt, insbesondere Methansulfonsäuremethylester.

Erfindungsgemäße inerte Nitroverbindungen sind Nitromethan, Nitroethan, Nitropropan und entsprechende strukturisomere Verbindungen und Gemische dieser Nitroverbindungen.

Geeignete Extraktionsmittel für das erfindungsgemäße Verfahren sind prinzipiell alle als Viskositätsmodertoren/Lösungsmittel aufgezählte Stoffklassen wie Nitroverbindungen, Sulfonsäuren und hiervon abgeleitete Ester, Carbonsäuren und hiervon abgeleitete Ester, Hydroxyisobuttersäuremethylester, sowie schlecht wasserlösliche Ketone, Ether und aromatische Lösungsmittel, insbesondere Methylketone der allgemeinen Formel R-C=O-R' (mit R = Me- und R' = C-1 bis C-12 Kohlenwasserstoffen), die verzweigt oder unverzweigt vorliegen können. Besonders bevorzugt sind Methylethylketon und Methylisobutylketon. Auch symmetrische und unsymmetrische Ketone können erfindungsgemäß als Lösungsmittel eingesetzt werden, wie zum Beispiel Diethylketon. Erfindungsgemäße aromatische Extraktionsmittel sind, zum Beispiel, Benzol, Toluol und Xylole.

Metallsalze, die als Katalysator der Dehydratisierung von HIBS zu MAS eingesetzt werden können, sind Alkali- und Erdalkalisalze, die in Lösung mit dem Edukt HIBS das entsprechende Metall-Hydroxyisobuttersäuresalz bilden. Hierbei sind die Alkali- und Erdalkalihydroxide bzw. Oxide, Carbonate und Hydrogencarbonate bevorzugt. Besonders bevorzugt sind Lithium-, Natrium-, Kalium- und Magnesium-, Calcium und Barium-Hydroxid, -Oxid und entsprechende Carbonate und Hydrogencarbonate.

Exemplarisch für die Alkohole die erfindungsgemäß im Verfahrensschritt f) eingesetzt werden können, sind C₁ bis C₁₂ Alkohole, insbesondere Methanol, Ethanol, Propanol und entsprechende homologe- und analoge Verbindungen bis C₁₂ haltiger Kohlenwasserstoffe.

Insgesamt verläuft das erfindungsgemäße Verfahren über folgende Prozessstufen:
a.) Amidierung:
   Herstellung einer Lösung bestehend im wesentlichen aus dem Hydrogensulfat-Salzes von alpha-Hydroxyisobuttersäureamid, dem Monoschwefelsäureester des alpha-Hydroxyisobuttersäureamids und eines Viskositätsmoderators/Lösungsmittels durch Umsetzung von Acetoncyanhydrin mit Schwefelsäure in Gegenwart von Wasser und dem Viskositätsmoderator/Lösungsmittel;
b.) Verseifung:
   Herstellung einer wässrigen Lösung von alpha-Hydroxyisobuttersäure, und Ammoniumhydrogensulfat durch Umsetzung der unter a.) hergestellten Lösung des Säureamids mit Wasser gegebenenfalls in Anwesenheit des unter a.) beschriebenen Lösungsmittels;
c.) Kreislaufführung des Lösungsmittels: Abtrennung des unter a.) verwendeten inerten Lösungsmittels vor oder nach Verseifung (Schritt b.) und Rückführung des Lösungsmittels/Viskositätsmoderators in die Prozessstufe a.);
d.) Isolierung von HIBS:
   Abtrennung des als Produkt gewünschten HIBS von der Prozesssäure durch Extraktion aus der nach den Prozessschritten b.) und c.) erhaltenen wässrigen,
   Ammoniumhydrogensulfat-haltigen HIBS-Lösung mit einem geeigneten organischen Lösungsmittel und anschließende Isolierung von HIBS durch Entfernung des Extraktionsmittels oder durch Kristallisation; und
e.) ß-Eliminierung:
   Herstellung von Methacrylsäure durch Umsetzung der unter e.) erhaltenen alpha-Hydroxyisobuttersäure in flüssiger Phase in Gegenwart eines Metallsalzes der alpha-Hydroxyisobuttersäure unter Wasserabspaltung (β-Eliminierung von Wasser).

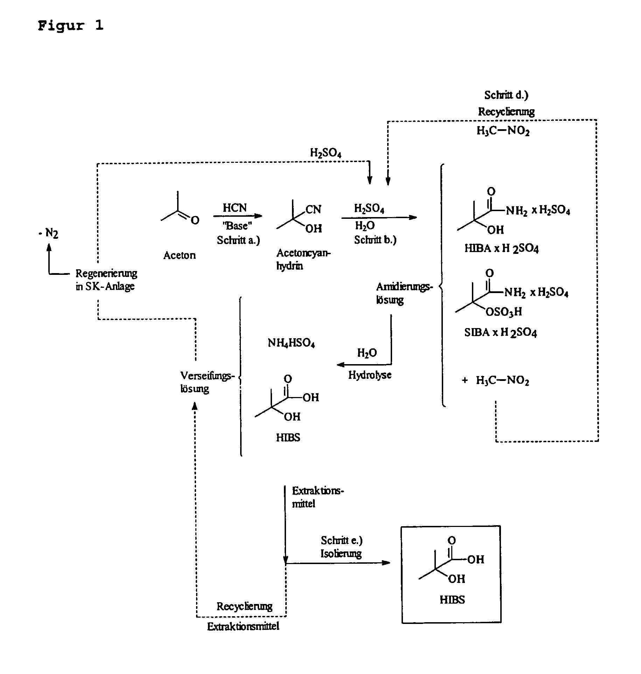

Figur 1 stellt die wesentlichen chemischen Reaktionen des erfindungsgemäßen Verfahrens mit Nitromethan als exemplarisches Lösungsmittel der Amidierung im Überblick schematisch dar.

Die vorliegende Erfindung wird im folgenden anhand von Ausführungsbeispielen näher erläutert.

### Vergleichsbeispiel A

Einige Beispiele aus JP Hei-4-193845 wurden nachgearbeitet. Dabei stellte man fest, dass bei Verwendung von nahezu stöchiometrischen Schwefelsäuremengen und Wassergehalten von ca. 0,1 Äquivalenten wie angegeben (siehe Beispiele 1-8 im Patent) die Viskosität selbst bei der maximalen Reaktionstemperatur gegen Ende der Reaktion nicht mehr beherrscht werden kann, der gesamte Ansatz verfestigte sich und es kam zum Bruch des eingesetzten mechanischen Rührers. Als Abhilfe wurde, im Laufe der Reaktion oder schon zu Beginn der ACH Dosierung, in Gegenwart von Methanol oder des Hydroxyisobuttersäuremethylesters gearbeitet, und so eine ausreichende Viskosität des Amidierungsgemisches sichergestellt.

Unter Variation der Parameter innerhalb der angegebenen Grenzen wurde festgestellt, dass unter diesen Bedingungen zwar die Viskosität des Amidierungsgemisches ausreichend niedrig ist (also Rührbarkeit des Reaktionsansatzes sichergestellt werden kann), aber die ACH Umsetzung nicht mehr vollständig ist (im Fall der Verwendung von Methanol), oder aber der zugesetzte Ester als Lösungsmittel (im Falle der Verwendung von HIBSM als Lösungsmittel) sich unter den Reaktionsbedingungen zersetzt.

Obwohl das Verfahren bezüglich der erreichbaren Esterausbeuten gut ist, machen die notwendigen Reaktionszeiten (mit einer Amidierungszeit von 2 Stunden und einer Veresterungszeit von 6 Stunden zum Erhalt guter Ausbeuten) das Verfahren außerordentlich unwirtschaftlich.

### Beispiel 1

### Herstellung von Hydroxyisobuttersäure in Gegenwart von Nitromethan

58,9 g einer 91,6 Gew.-%igen H₂SO₄ (enthält als 100 Gew.-% 53,95 g oder 550 mmol Schwefelsäure) werden unter Rühren ohne Stabilisator mit 30 g Nitromethan (techn.) versetzt. Die farblose Lösung enthält damit 4,95 g Wasser (= 0,275 mol). Diese Lösung wird in einem 250 ml Dreihalskolben bei 40°C vorgelegt. Mittels einer HPLC Pumpe werden zu dieser "wässrigen" Schwefelsäure unter mechanischer Rührung 0,5 Mol ACH ( = 42,6 g) mit einer Förderrate von 2,3 ml/min gegeben, wobei die deutliche Exotherme während der Reaktion mit einem Wasserbad abgefangen wird (d.h. Reaktionstemperatur: 40°C). Während des ersten Teils der ACH Zugabe (0 - 15 min ACH Zugabe) arbeitet man bei 40°C; die Reaktionslösung ist hierbei klar und sehr wenig viskos.

Während des zweiten Teils der ACH Zugabe (d.h. ab der 15ten Minute bis Minute 20 der ACH Zugabe) arbeitet man bei 45°C; die Reaktionslösung ist hierbei klar und etwas viskoser, aber sehr gut rührbar. Die Gasentwicklung ist im Vergleich zu allen anderen Verfahrensweisen minimal, die Rührbarkeit während der gesamten Amidierungszeit sehr gut.

Die Gesamtdosierdauer beträgt genau 20 min, danach folgt eine Nachreaktionszeit von 10 min bei 55°C. Damit beträgt das molare Verhältnis ACH/Schwefelsäure/Wasser = 1:1,1:0,55.

Man erhält 131,3 g Auswaage (Theorie = 131,5 g) was einer quantitativen Wiederfindung entspricht. Nur minimale Gasentwicklung wird beobachtet. Nach Ablauf der Amidierung wird zur zähen Reaktionslösung schnell unter Kühlung (Temperatur etwa 50°C - 60°C) 85,05 g Wasser zugegeben. Das entspricht einer Stöchiometrie von HIBA/Schwefelsäure/Wasser von 1:1,1:10.

Diese Lösung wird 1 h lang in der Schottflasche (unter moderatem Druck, ca. 2,5 bar_{abs}.) im Ölbad auf 120°C erwärmt, wobei sich HIBA komplett zu HIBS umsetzt. Man erhält bei Reaktionsende eine farblose Lösung von 213,9 g, das sich leicht rühren lässt (wasserklare Lösung sehr niedrige Viskosität), die laut HPLC 23,7 Gew.-% Hydroxyisobuttersäure (50,7 g = 0,487 mol HIBS = 98,0% der Theorie bzgl. ACH) enthält. Als weiteres mit HPLC zu detektierendes Nebenprodukt wird Aceton gefunden (0,16 Gew.-%, d.h. ca. 1,0% der Theorie bezüglich ACH).

Im einfachsten Fall kann damit ACH mit einem leichten Überschuss wässriger Schwefelsäure (in Gegenwart von Nitromethan als Viskositätsmoderator der Amidierung) bei moderaten Temperaturen (40°C - 55°C) zunächst zu einer HIBA x H₂SO_{4/}H₂SO₄ Mischung umgesetzt werden, die im zweiten Schritt durch Umsetzung mit Wasser selektiv zum HIBS verseift wird.

Nitromethan kann nach der Hydrolyse durch Entfernung des Azeotrops mit Wasser entfernt und in die Amidierung zurückgeführt werden.

### Beispiel 2

### Herstellung von Hydroxyisobuttersäure in Gegenwart von Essigsäure

56,5 g einer 95,55 Gew.-%igen H₂SO₄ (enthält als 100 Gew.-% 53,95 g oder 550 mmol) werden unter Rühren mit 100 mg Hydrochinon versetzt, das langsam in Lösung geht. Die farblose Lösung enthält damit 2,55 g Wasser (= 0,142 mol). Diese Lösung wird in einem 250 ml Dreihalskolben bei 40°C vorgelegt. Mittels einer HPLC Pumpe werden zu dieser "wässrigen" Schwefelsäure unter mechanischer Rührung 0,5 Mol ACH (= 42,6 g) mit einer Förderrate von 2,3 ml/min gegeben, wobei die deutliche Exotherme während der Reaktion mit einem Wasserbad abgefangen wird (d.h. Reaktionstemperatur: 40°C -45°C). Während der ersten Hälfte der ACH Zugabe (d.h. 0 - 10 min ACH Zugabe) ist die Reaktionslösung klar und von einer honigartigen Konsistenz, nach etwa 10 min treten dann erste, fein verteilte Gasblasen auf, die auf ACH Fragmentierung zu CO schließen lassen.

Nach zehn Minuten tropft man über einen separaten Tropftrichter während 4-8 min 30 g Essigsäure zur Reaktionslösung hinzu, während die ACH Dosierung fortgesetzt wird.

Die Gesamtdosierdauer beträgt genau 20 min, danach folgt eine Nachreaktionszeit von 40 min bei 55°C. Damit beträgt das molare Verhältnis ACH/Schwefelsäure/Wasser = 1:1,1:0,28.

Man erhält 129,1 g Auswaage (Theorie = 129,1 g) was einer quantitativen Wiederfindung entspricht. Nur minimale Gasentwicklung wird beobachtet. Nach Ablauf der Amidierung wird zur zähen Reaktionslösung schnell unter Kühlung (Temperatur etwa 50°C - 60°C) 87,5 g Wasser zugegeben. Das entspricht einer Stöchiometrie von HIBA/Schwefelsäure/Wasser von 1:1,1:10.

Diese Lösung wird 1 h lang in der Schottflasche (unter moderatem Druck) im Ölbad auf 130°C erwärmt, wobei sich HIBA komplett zu HIBS umsetzt. Nach 30 min und 60 min wird jeweils eine Probe genommen, um den Verlauf der Reaktion zu verfolgen.

Man erhält bei Reaktionsende eine farblose Lösung von 214,7 g, das sich leicht rühren lässt (wasserklare Lösung sehr niedrige Viskosität), die laut HPLC nach 30 min bereits 23,95 Gew.-% Hydroxyisobuttersäure (51,42 g = 0,493 mol HIBS = 98,8% der Theorie bzgl. ACH). Nach 1 h werden noch 23,90 Gew.-% HIBS detektiert. Als einziges zu detektierendes Nebenprodukt wird Aceton gefunden (0,15 Gew.-%, d.h. ca. 1% der Theorie bezüglich ACH).

Im einfachsten Fall kann damit ACH mit einem leichten Überschuss wässriger Schwefelsäure (in Gegenwart von Essigsäure als Viskositätsmoderator der Amidierung) bei moderaten Temperaturen (40°C - 60°C) zunächst zu einer HIBA x H₂SO_{4/}H₂SO₄ Mischung umgesetzt werden, die im zweiten Schritt durch Umsetzung mit Wasser selektiv zum HIBS verseift wird.

### Beispiel 3

### Herstellung von Hydroxyisobuttersäuremethylester in Gegenwart von Essigsäure

53,5 g einer 96,2 Gew.-%igen H₂SO₄ (enthält als 100 Gew.-% 51,5 g oder 525 mmol) werden Hydrochinon-frei vorgelegt. Die farblose Lösung enthält damit 2,0 g Wasser (= 0,11 mol oder 22 mol% bezüglich ACH). Hierzu werden unter Rühren und Abfangen einer leichten Exotherme 30 g Essigsäure zugegeben (= 0,5 Mol). Diese Lösung wird in einem 250 ml Dreihalskolben bei 40°C vorgelegt. Mittels Tropftrichter werden zu dieser "wässrigen" Schwefelsäure-AcOH Lösung unter Zugabe 0,5 Mol ACH (= 42,6 g) mit einer Förderrate von ca. 2,3 ml/min gegeben, wobei die deutliche Exotherme während der Reaktion mit einem Wasserbad abgefangen wird (d.h. Reaktionstemperatur: 45°C -50°C). Während der ersten Hälfte der ACH Zugabe (d.h. 0 - 10 min ACH Zugabe) ist die Reaktionslösung klar und leicht rührbar, nach etwa 10 min treten dann wenig erste, fein verteilte Gasblasen auf, die auf ACH Fragmentierung zu CO schließen lassen.

Die Gesamtdosierdauer beträgt genau 20 min, danach folgt eine Nachreaktionszeit von 40 min bei 55°C. Damit beträgt das molare Verhältnis ACH/Schwefelsäure/Wasser = 1:1,05:0,22.

Man erhält 126,2 g Auswaage (Theorie = 126,1 g) was einer quantitativen Wiederfindung entspricht. Nur minimale Gasentwicklung wird beobachtet. Nach Ablauf der Amidierung wird zur zähen Reaktionslösung schnell unter Kühlung (Temperatur etwa 50°C) 11,5 g Wasser und 4 Äquivalente Methanol (64 g MeOH) zugegeben. Das entspricht einer Stöchiometrie von HIBA/Schwefelsäure/Wasser/MeOH von 1:1,05:1,5:4.

Diese Lösung wird 1 h lang in der Schottflasche (unter moderatem Druck) im Ölbad auf 115°C erwärmt, wobei sich HIBA komplett zu HIBS-HIBSM umsetzt. Nach 60 min wird jeweils eine Probe genommen, um den Verlauf der Reaktion zu verfolgen.

Man erhält bei Reaktionsende eine farblose zweiphasige Lösung von 201,8 g, die sich leicht rühren lässt (wasserklare Lösung sehr niedriger Viskosität), füllt mit Wasser auf insgesamt 400 g auf und erhält so eine einphasige Lösung, die laut HPLC nach 60 min 2,5 Gew.-% Hydroxyisobuttersäure (10,0 g = 0,0962 mol HIBS = 19,2% der Theorie bzgl. ACH) enthält, des weiteren werden noch 12,0 Gew.-% HIBSM (= 48,0 g = 0,40 mol = 81,5%) detektiert. Als einziges Produkt wird laut HPLC-Methode Methylacetat detektiert (7,4 Gew.-%, d.h. 29,6 g = 0,40 mol MeAc, also 80% bezüglich Essigsäure).

Die Gesamtausbeute an HIBS + HIBSM beträgt also bei dieser Reaktionsführung 100,7% bezüglich ACH (ist also quantitativ).

Im einfachsten Fall kann damit ACH mit einem leichten Überschuss wässriger Schwefelsäure (in Gegenwart von Essigsäure als Viskositätsmoderator der Amidierung) bei moderaten Temperaturen (40°C - 60°C) zunächst zu einer HIBA x H₂SO_{4/}H₂SO₄ Mischung umgesetzt werden, die im zweiten Schritt durch Umsetzung mit Wasser/Methanol selektiv zu einer Mischung aus HIBS und HIBSM (Verhältnis ca. 1:4) verseift/verestert wird.

Die Zweiphasigkeit der nach Reaktion erhaltenen Lösung ergibt sich durch die Anwesenheit von Methylacetat, das sich einwandfrei von der Salzlösung abtrennt und gleichzeitig HIBSM extrahiert.

### Beispiel 4

### Herstellung von Hydroxyisobuttersäure in Gegenwart von Methansulfonsäure

56,5 g einer 95,55 Gew.-%igen H₂SO₄ (enthält als 100 Gew.-% 53,95 g oder 550 mmol) werden unter Rühren ohne Hydrochinon vorgelegt. Die farblose Lösung enthält damit 2,55 g Wasser (= 0,142 mol). Diese Lösung wird in einem 250 ml Dreihalskolben bei 40°C eingestellt. Zu dieser Lösung werden als Viskositätsmoderator 30 g Methansulfonsäure gegeben, die ohne merkliche Exotherme eintropfbar sind.

Mittels einer HPLC Pumpe werden zu dieser "wässrigen" Schwefelsäure-Methansulfonsäure Lösung unter mechanischer Rührung 0,5 Mol ACH (= 42,6 g) mit einer Förderrate von 2,3 ml/min gegeben, wobei die deutliche Exotherme während der Reaktion mit einem Wasserbad abgefangen wird (d.h. Reaktionstemperatur: 40°C -45°C). Während der ersten Hälfte der ACH Zugabe (d.h. 0 - 10 min ACH Zugabe) ist die Reaktionslösung klar und von einer Konsistenz wie Honig, nach etwa 10 min treten dann erste, fein verteilte Gasblasen auf, die auf sehr geringe ACH Fragmentierung zu CO schließen lassen.

Die Gesamtdosierdauer beträgt genau 20 min, danach folgt eine Nachreaktionszeit von 40 min bei 55°C. Damit beträgt das molare Verhältnis ACH/Schwefelsäure/Wasser = 1:1,1:0,28.

Man erhält 129,1 g Auswaage (Theorie = 129,1 g) was einer quantitativen Wiederfindung entspricht. Nur minimale Gasentwicklung wird beobachtet. Nach Ablauf der Amidierung wird zur zähen Reaktionslösung schnell unter Kühlung (Temperatur etwa 50°C - 60°C) 87,5 g Wasser zugegeben. Das entspricht einer Stöchiometrie von HIBA/Schwefelsäure/Wasser von 1:1,1:10.

Diese Lösung wird 1 h lang in der Schottflasche (unter moderatem Druck) im Ölbad auf 130°C erwärmt, wobei sich HIBA komplett zu HIBS umsetzt. Nach 30 min und 60 min wird jeweils eine Probe genommen, um den Verlauf der Reaktion zu verfolgen.

Man erhält bei Reaktionsende eine farblose Lösung von 215,2 g, das sich leicht rühren lässt (wasserklare Lösung sehr niedrige Viskosität), die laut HPLC nach 60 min bereits 24,0 Gew.-% Hydroxyisobuttersäure (51,7 g = 0,497 mol HIBS = 99,3% der Theorie bzgl. ACH) enthält. Als einziges zu detektierendes Nebenprodukt wird Aceton gefunden (0,3 Gew.-%, d.h. 0,65 g oder 1,1 mmol oder 0,3% der Theorie bezüglich ACH).

Im einfachsten Fall kann damit ACH mit einem leichten Überschuss wässriger Schwefelsäure (in Gegenwart von Methansulfonsäure (MS) als Viskositätsmoderator der Amidierung) bei moderaten Temperaturen (40°C - 60°C) zunächst zu einer HIBA x H₂SO_{4/}H₂SO₄ Mischung umgesetzt werden, die im zweiten Schritt durch Umsetzung mit Wasser selektiv zum HIBS verseift wird.

Die Ergebnisse der Beispiele 1 und 2 sind in Tabelle 1 zusammengefasst:

**Tabelle 1**

| Beispiel Nr. | H₂SO₄-Konz. (Gew.-%) | Molverhältnis Amidierung ACH-H₂O-H₂SO₄ | Reaktionstemp; Dauer Amidierung [°C]/min | Molverhält. Verseifung ACH-H₂O-H₂SO₄-LM * | Reaktionstemp. Dauer Hydrolyse [°C]/min | ACH-Umsatz | HIBS/HIBSM Ausbeute |
|---|---|---|---|---|---|---|---|
| 1 | 91,6 | 1/0,55/1,1 + 60 g Nitromethan | 40-55°C/30 min (15 min bei 40°C, 5 min bei 55°C, 10 min bei 55°C Nachreaktion) | 1/10/1,1 +60 g Nitromethan | 120°C/60 min | >99% | 98,0% HIBS 1,2% Aceton |
| 2 | 95,6 | 1/0,3/1,1 + 1,0 mol Essigsäure | Stufe 1: 10 min bei 40°C, Stufe 2: 10 min bei 55°C, Nachreakt.: 40 min 55°C | 1/10/1,1/1,0 Essigsäure | 130°C/30 min | >99% | 98,8 HIBS 1,0% Aceton kein MMA |

| | | | | | | | |
|---|---|---|---|---|---|---|---|
| * LM = Lösungsmittel | | | | | | | |

### Beispiele 5-10

### Herstellung von Hydroxyisobuttersäure ex ACH in Gegenwart von Nitromethan mit verschiedenen Wassergehalten und Hydrolyseparametern

Der Standardansatz bestand aus wässrige Schwefelsäure und Nitromethan gemischt und vorgelegt im Dreihalskolben bei ca. 35°C, dazu unter Kühlung 0,5 mol ACH mit 2,3 ml/min per HPLC-Pumpe (wenn nicht anders gekennzeichnet) eindosiert, anschließend entsprechend Tabelleneintrag nachreagiert. Anschließend wurde Wasser zugegeben und in der Schottflasche ohne Rühren hydrolisiert. Ausbeutebestimmung nach Hydrolyse mit Wasser durch HPLC-Quantifizierung (gegen externen Standard) von HIBS und Aceton in der Rohlösung. Die Ergebnisse dieser Beispiele 5-10 sind in Tabelle 2 dargestellt.

**Tabelle 2**

| Beispiel Nr. | H₂SO₄-Konz. (Gew.-%) | Molverhältnis Amidierung ACH-H₂O-H₂SO₄ | Reaktionstemp; Dauer Amidierung [°C]/min | Molverhält. Verseifung ACH-H₂O-H₂SO₄-LM * | Reaktionstemp; Dauer Hydrolyse [°C]/min | ACH-Umsatz | HIBS Ausbeute |
|---|---|---|---|---|---|---|---|
| 5 | 91,6% | 1/0,6/1,1 + 60 g Nitromethan | 40-55°C/30 min (15 min bei 40°C, 5 min bei 55°C, 10 min bei 55°C Nachreaktion) | 1/10/1,1 + 60 g Nitromethan | 120°C/60 min | > 99% | 98,0% HIBS 1,2% Aceton |
| 6 | 93,0% | 1/0,45/1,1 + 100 g Nitromethan | 40-55°C/30 min (15 min bei 40°C, 5 min bei 55°C, 10 min bei 55°C) | 1/10/1,1 + 60 g Nitromethan | 120°C/60 min | > 99% | 99,2% HIBS 0,8% Aceton |
| 7 | 91,5% | 1/0,53/1,05 + 60 g Nitromethan | 40-50°C/30 min (15 min bei 45°C, 5 min bei 50°C, 10 min bei 60°C) | 1/10/1,05 + 60 g Nitromethan | 120°C/60 min | > 99% | 98,4% HIBS n.b.** % Aceton |
| 8 | 92,9% | 1/0,42/1,0 + 60 g Nitromethan | 40-55°C/30 min (15 min bei 40°C, 5 min bei 55°C, 10 min bei 55°C) | 1/6/1,0 + 60 g Nitromethan | 120°C/60 min | > 99% | 98,7% HIBS 1,2% Aceton |
| 9 | 92,9% | 1/0,42/1,0 + 60 g Nitromethan | 40-55°C/30 min (15 min bei 40°C, 5 min bei 55°C, 10 min bei 55°C) | 1/10/1,0 + 60 g Nitromethan | 120°C/60 min | > 99% | 98,8% HIBS 1,2% Aceton |
| 10 | 92,9% | 1/0,43/1,05 + 60 g Nitromethan | 40-55°C/15 min (7,5 min bei 43°C, 2,5 min bei 55°C, 5 min bei 55°C) | 1/5/1,05 + 60 g Nitromethan | 130°C/30 min | > 99% | 98,6% HIBS 1,3% Aceton |

| | | | | | | | |
|---|---|---|---|---|---|---|---|
| * LM = Lösungsmittel ** n.b. = nicht berechnet | | | | | | | |

### Beispiele 11-18

### Herstellung von Hydroxyisobuttersäure ex ACH in Gegenwart von diversen Viskositätsmoderatoren

Der Standardansatz bestand aus wässrige Schwefelsäure und Nitromethan gemischt und vorgelegt im Dreihalskolben bei ca. 35°C, dazu unter Kühlung 0,5 mol ACH mit 2,3 ml/min per HPLC-Pumpe (wenn nicht anders gekennzeichnet) eindosiert, anschließend wurde entsprechend Tabelleneintrag nachreagiert. Anschließend wurde Wasser zugegeben und in der Schottflasche ohne Rühren hydrolisiert; Ausbeutebestimmung nach Hydrolyse mit Wasser durch HPLC-Quantifizierung (gegen externen Standard) von HIBS und Aceton in der Rohlösung. Die Viskositätsmoderatoren wurden ausgewählt aus der Gruppe von Nitroalkanen, Sulfonsäureester, Hydroxyisobuttersäuremethylester und Carbonsäuren (exemplarisch Isobuttersäure). Die Ergebnisse dieser Beispiele 11-18 sind in Tabelle 3 zusammengefasst:

**Tabelle 3**

| Beispiel Nr. | H₂SO₄-Konz. (Gew.-%) | Molverhältnis Amidierung ACH-H₂O-H₂SO₄ | Reaktionstemp; Dauer Amidierung [°C]/min | Molverhält. Verseifung ACH-H₂O-H₂SO₄-LM * | Reaktionstemp; Dauer Hydrolyse [°C]/min | ACH-Umsatz | HIBS Ausbeute |
|---|---|---|---|---|---|---|---|
| 11 | 91,6% | 1/0,6/1,1 + 60 g Nitroethan | 40-55°C/20 min (10 min bei 40°C, 5 min bei 55°C, 5 min bei 55°C Nachreaktion) | 1/10/1,1 (enthält 60 g Nitroethan) | 120°C/45min | > 99% | 99,0% HIBS 0,8% Aceton |
| 12 | 93,0% | 1/0,45/1,1 + 100 g Nitropropan | 40-55°C/30 min (15 min bei 40°C, 5 min bei 55°C, 10 min bei 55°C) | 1/10/1,1 (enthält 100 g Nitropropan) | 130°C/60 min | > 99% | 99,2% HIBS 0,8% Aceton |
| 13 | 91,5% | 1/0,53/1,05 + 60 g Nitroethan | 40-50°C/20 min (10 min bei 45°C, 5 min bei 50°C, 10 min bei 60°C) | 1/6/1,05 + 60 g Nitroethan | 130°C/60 min | > 99% | 98,0% HIBS 1,4% Aceton |
| 14 | 92,9% | 1,0/0,42/1,0 + 30 g Nitromethan | 40-55°C/30 min (15 min bei 40°C, 5 min bei 55°C, 10 min bei 55°C) | 1/6/1,0 + 60 g Nitromethan | 120°C/60 min | > 99% | 98,9% HIBS 1,0% Aceton |
| 15 | 92,9% | 1/0,38/0,9 + 30 g Nitromethan | 40-55°C/30 min (15 min bei 40°C, 5 min bei 55°C, 10 min bei 55°C) | 1/10/0,9 (enthält 30 g Nitromethan) | 120°C/60 min | > 99% | 97,9% HIBS 1,9% Aceton |
| 16 | 92,9% | 1/0,38/0,9 + 30 g Nitromethan | 40-55°C/15 min (10 min bei 40°C, 10 min bei 55°C, 5 min bei 55°C) | 1/6/0,9 (enthält 30 g Nitromethan) | 130°C/60 min | > 99% | 98,0% HIBS 1,7% Aceton |
| 17 | 93,1% | 1/0,41/1,0 + 30 g Nitromethan | 40-55°C/20 min (10 min bei 40°C, 5 min bei 55°C) | 1/7,1/1,0 (enthält 30 g Nitromethan) | 130°C/30 min | > 99% | 99,0% HIBS 0,5% Aceton |
| 18 | 93,1% | 1/0,41/1,0 + 50 g Methansulfon-säuremethyl-ester | 40-55°C/20 min (10 min bei 40°C, 5 min bei 55°C) | 1/6,0/1,0 (enthält 50 g Sulfonsäure-ester) | 130°C/60 min | > 99% | 98,0% HIBS 1,1% Aceton |

| | | | | | | | |
|---|---|---|---|---|---|---|---|
| * LM = Lösungsmittel | | | | | | | |

## Patentansprüche

1. Verfahren zur Herstellung von Methacrylsäure, **dadurch gekennzeichnet, dass** man
a) Acetoncyanhydrin bei Temperaturen unter 80°C mit maximal 1,2 Äquivalenten Schwefelsäure in Gegenwart von 0,05 - 1,0 Äquivalenten Wasser in Gegenwart eines unter den Reaktionsbedingungen inerten, polaren Lösungsmittels, welches im Kreis geführt wird, unter Entstehung einer gut rührbaren Lösung der entsprechenden Amidsulfate im inerten Lösungsmittel umsetzt,
b) nach Zugabe von Wasser diese Lösung in Gegenwart oder nach vorheriger Entfernung des inerten Lösungsmittels zu einer Lösung bestehend im wesentlichen aus Wasser, Ammoniumhydrogensulfat und alpha-Hydroxyisobuttersäure umsetzt,
c) Hydroxyisobuttersäure durch Extraktion mit einem geeigneten Extraktionsmittel von der wässrigen Ammoniumhydrogensulfatlösung abtrennt,
d) nach Entfernung des Extraktionsmittels die in hoher Konzentration gewonnene alpha-Hydroxyisobuttersäure in Gegenwart eines Metallsalzes der alpha-Hydroxyisobuttersäure bei Temperaturen zwischen 160 - 300°C in flüssiger Phase zu einem als Destillat anfallenden Gemisch bestehend aus im wesentlichen Methacrylsäure und Wasser umsetzt, und
e) Methacrylsäure destillativ aus diesem Gemisch in hochreiner Form gewinnt oder das unter d) anfallende Produktgemisch (MAS-Wasser) als Extraktionsmittel für die Isolierung der alpha-Hydroxyisobuttersäure nach Schritt c) einsetzt und anschließend die Wertstoffe destillativ voneinander trennt.

2. Verfahren gemäß Anspruch 1, **dadurch gekennzeichnet, dass** der Verfahrensschritt a) bei einer Temperatur kleiner als 70°C ausgeführt wird.

3. Verfahren gemäß Anspruch 1 oder 2, **dadurch gekennzeichnet, dass** als Lösungsmittel eine inerte C₂-C₁₂ Carbonsäure, inerte Nitroverbindung oder eine aliphatische Sulfonsäure eingesetzt wird.

4. Verfahren gemäß Anspruch 3, **dadurch gekennzeichnet, dass** die inerte C₂-C₁₂ Carbonsäure eine Carbonsäure ausgewählt aus der Gruppe von Essigsäure, Propionsäure, Methylpropansäure, Buttersäure, Isobuttersäure und entsprechende homologe höherkettige, aliphatische verzweigte und unverzweigte Carbonsäure ist.

5. Verfahren gemäß Anspruch 4, **dadurch gekennzeichnet, dass** die inerte C₂-C₁₂ Carbonsäure Essigsäure ist.

6. Verfahren gemäß Anspruch 3, **dadurch gekennzeichnet, dass** die inerte Nitroverbindung Nitromethan ist.

7. Verfahren gemäß Anspruch 3, **dadurch gekennzeichnet, dass** die aliphatische Sulfonsäure Methansulfonsäure ist.

8. Verfahren gemäß Anspruch 1, **dadurch gekennzeichnet, dass** man in Schritt c) Hydroxyisobuttersäure durch Extraktion mit einem Extraktionsmittel von der wässrigen Ammoniumhydrogensulfatlösung trennt und diese wässrige Ammoniumhydrogensulfat in einer Schwefelsäurekontaktanlage unter Entstehung von Stickstoff zu Schwefelsäure umarbeitet, die gemäß Schritt a) in die Amidierung zurückgeführt kann.

9. Verfahren gemäß Anspruch 8, **dadurch gekennzeichnet, dass** als Extrationsmittel Nitroverbindungen, Sulfonsäuren und davon abgeleitete Ester, Carbonsäuren und davon abgeleitete Ester, Hydroxyisobuttersäuremethylester, sowie schlecht wasserlösliche Ketone, Ether oder aromatische Lösungsmittel, der allgemeinen Formel R-C=O-R' (mit R = Me- und R' = C-1 bis C-12 Kohlenwasserstoffen, die verzweigt oder unverzweigt eingesetzt werden.

10. Verfahren gemäß Anspruch 8 oder 9, **dadurch gekennzeichnet, dass** als Extraktionsmittel Methylethylketon oder Methylisobutylketon eingesetzt werden.

11. Verfahren gemäß einem oder mehreren der vorhergehenden Ansprüche, **dadurch gekennzeichnet, dass** man einen zusätzlichen Schritt f) wie folgt ausführt:
f) das unter Schritt e) erhaltene Gemisch aus Methacrylsäure/Wasser oder reiner Methacrylsäure selbst wird mit einem Alkohol umgesetzt, wobei der als Produkt gewünschte Ester (= Methacrylsäureester) in einer für die Anwendung gewünschte Form nach bekannten Methoden gewonnen wird.

12. Verfahren gemäß Anspruch 11, **dadurch gekennzeichnet, dass** der Alkohol Methanol, Ethanol, Propanol und entsprechende homologe- und analoge Verbindungen bis C₁₂ haltiger Kohlenwasserstoffe ist.

13. Verfahren gemäß einem oder mehreren der vorhergehenden Ansprüche 1 bis 12, **dadurch gekennzeichnet, dass** bei einer Reaktionszeit für die Amidierung unter 60 min, und bei einer Reaktionszeit für die Hydrolyse unter 120 min, vollständige Umsätze von größer als (>) 99% erzielt werden.

14. Verfahren gemäß einem oder mehreren der vorhergehenden Ansprüche 1 bis 12, **dadurch gekennzeichnet, dass** bei einer Reaktionszeit für die Amidierung unter 30 min, und bei einer Reaktionszeit für die Hydrolyse unter 100 min umgesetzt wird.

15. Verfahren gemäß einem oder mehreren der vorhergehenden Ansprüche 1 bis 12, **dadurch gekennzeichnet, dass** bei einer Reaktionszeit für die Amidierung unter 20 min, und bei einer Reaktionszeit für die Hydrolyse unter 75 min umgesetzt wird.

## Claims

1. Process for preparing methacrylic acid, **characterized in that**
a) acetone cyanohydrin is reacted at temperatures below 80°C with a maximum of 1.2 equivalents of sulphuric acid in the presence of 0.05-1.0 equivalent of water in the presence of a polar solvent which is circulated and inert under the reaction conditions to form an efficiently stirrable solution of the corresponding amide sulphates in the inert solvent,
b) after adding water, this solution, in the presence of or after preceding removal of the inert solvent, is converted to a solution consisting substantially of water, ammonium hydrogensulphate and alpha-hydroxyisobutyric acid,
c) hydroxyisobutyric acid is removed from the aqueous ammonium hydrogensulphate solution by extraction with a suitable extractant,
d) after removing the extractant, the alpha-hydroxyisobutyric acid obtained in high concentration, in the presence of a metal salt of the alpha-hydroxyisobutyric acid, is converted at temperatures between 160-300°C in the liquid phase to a mixture obtained as a distillate and consisting substantially of methacrylic acid and water, and
e) methacrylic acid is obtained distillatively in highly pure form from this mixture or the product mixture obtained under d) (MAA water) is used as an extractant for the isolation of the alpha-hydroxyisobutyric acid in step c) and the materials of value are subsequently distillatively separated from one another.

2. Process according to Claim 1, **characterized in that** process step a) is performed at a temperature of less than 70°C.

3. Process according to Claim 1 or 2, **characterized in that** the solvent used is an inert C₂-C₁₂ carboxylic acid, inert nitro compound or an aliphatic sulphonic acid.

4. Process according to Claim 3, **characterized in that** the inert C₂-C₁₂ carboxylic acid is a carboxylic acid selected from the group of acetic acid, propionic acid, methylpropanoic acid, butyric acid, isobutyric acid and corresponding homologous longer-chain aliphatically branched and unbranched carboxylic acid.

5. Process according to Claim 4, **characterized in that** the inert C₂-C₁₂ carboxylic acid is acetic acid.

6. Process according to Claim 3, **characterized in that** the inert nitro compound is nitromethane.

7. Process according to Claim 3, **characterized in that** the aliphatic sulphonic acid is methanesulphonic acid.

8. Process according to Claim 1, **characterized in that**, in step c), hydroxyisobutyric acid is separated from the aqueous ammonium hydrogensulphate solution by extraction with an extractant and this aqueous ammonium hydrogensulphate solution is converted in a sulphuric acid contact plant with formation of nitrogen to sulphuric acid which can be recycled into the amidation in step a).

9. Process according to Claim 8, **characterized in that** the extractants used are nitro compounds, sulphonic acids and esters derived therefrom, carboxylic acids and esters derived therefrom, methyl hydroxyisobutyrate, and sparingly watersoluble ketones, ethers or aromatic solvents of the general formula R-C=O-R' (where R = Me- and R' = C-1 to C-12 hydrocarbons) which may be branched or unbranched.

10. Process according to Claim 8 or 9, **characterized in that** the extractants used are methyl ethyl ketone or methyl isobutyl ketone.

11. Process according to one or more of the preceding claims, **characterized in that** an additional step f) is performed as follows:
f) the mixture, obtained under step e), of methacrylic acid/water or pure methacrylic acid itself is reacted with an alcohol to obtain the ester desired as the product (= methacrylic ester) in a form desired for the application by known methods.

12. Process according to Claim 11, **characterized in that** the alcohol is methanol, ethanol, propanol and corresponding homologous and analogous compounds up to C₁₂ hydrocarbons.

13. Process according to one or more of the preceding Claims 1 to 12, **characterized in that** full conversions of greater than (>) 99% are achieved at a reaction time for the amidation of below 60 min, and at a reaction time for the hydrolysis of below 120 min.

14. Process according to one or more of the preceding Claims 1 to 12, **characterized in that** the reaction is effected at a reaction time for the amidation of below 30 min, and at a reaction time for the hydrolysis of below 100 min.

15. Process according to one or more of the preceding Claims 1 to 12, **characterized in that** the reaction is effected at a reaction time for the amidation of below 20 min, and at a reaction time for the hydrolysis of below 75 min.

## Revendications

1. Procédé pour la production d'acide méthacrylique, **caractérisé en ce que**
a) on fait réagir de l'acétonecyanhydrine à des températures inférieures à 80 °C avec au maximum 1,2 équivalent d'acide sulfurique en présence de 0,05 - 1,0 équivalent d'eau en présence d'un solvant polaire inerte dans les conditions réactionnelles, qui est mis en circuit, avec obtention d'une solution bien agitable des amide-sulfates correspondants dans un solvant inerte,
b) après addition d'eau on convertit cette solution en présence ou après élimination préalable du solvant inerte en une solution consistant essentiellement en eau, hydrogénosulfate d'ammonium et acide alpha-hydroxyisobutyrique,
c) on sépare l'acide hydroxyisobutyrique de la solution aqueuse d'hydrogénosulfate d'ammonium par extraction à l'aide d'un agent d'extraction approprié,
d) après élimination de l'agent d'extraction on convertit l'acide alpha-hydroxyisobutyrique, obtenu à forte concentration, en présence d'un sel métallique de l'acide alpha-hydroxyisobutyrique à des températures comprises entre 160 et 300 °C en phase liquide, en un mélange formé en tant que distillat, consistant essentiellement en acide méthacrylique et eau, et
e) à partir de ce mélange on obtient par distillation de l'acide méthacrylique sous forme très pure ou on utilise le mélange de produits (AMA-eau) formé en d) comme agent d'extraction pour l'isolement de l'acide alpha-hydroxyisobutyrique selon l'étape c) et ensuite on sépare les unes des autres par distillation les substances de valeur.

2. Procédé selon la revendication 1, **caractérisé en ce que** l'étape a) du procédé est effectuée à une température inférieure à 70 °C.

3. Procédé selon la revendication 1 ou 2, **caractérisé en ce qu'**on utilise comme solvant un acide carboxylique en C₂-C₁₂ inerte, un composé nitré inerte ou un acide sulfonique aliphatique.

4. Procédé selon la revendication 3, **caractérisé en ce que** l'acide carboxylique en C₂-C₁₂ inerte est un acide carboxylique choisi dans le groupe constitué par l'acide acétique, l'acide propionique, l'acide méthylpropanoïque, l'acide butyrique, l'acide isobutyrique et un acide carboxylique homologue à plus longue chaîne, aliphatique, ramifié ou non ramifié, correspondant.

5. Procédé selon la revendication 4, **caractérisé en ce que** l'acide carboxylique en C₂-C₁₂ inerte est l'acide acétique.

6. Procédé selon la revendication 3, **caractérisé en ce que** le composé nitré inerte est le nitrométhane.

7. Procédé selon la revendication 3, **caractérisé en ce que** l'acide sulfonique aliphatique est l'acide méthanesulfonique.

8. Procédé selon la revendication 1, **caractérisé en ce que** dans l'étape c) on sépare par extraction à l'aide d'un agent d'extraction l'acide hydroxyisobutyrique de la solution aqueuse d'hydrogénosulfate d'ammonium et on reconvertit cet hydrogénosulfate d'ammonium aqueux dans une unité de contact d'acide sulfurique, avec formation d'azote, en acide sulfurique, qui peut être renvoyé dans l'amidation selon l'étape a).

9. Procédé selon la revendication 8, **caractérisé en ce qu'**on utilise comme agent d'extraction des composés nitrés, des acides sulfoniques et des esters dérivés de ceux-ci, des acides carboxyliques et des esters dérivés de ceux-ci, l'hydroxyisobutyrate de méthyle, ainsi que des cétones difficilement solubles, des éthers ou des solvants aromatiques, de formule générale R-C=O-R' (où R = Me- et R' = hydrocarbures en C₁-C₁₂), qui peuvent être ramifiés ou non ramifiés.

10. Procédé selon la revendication 8 ou 9, **caractérisé en ce qu'**on utilise comme agent d'extraction la méthyléthylcétone ou la méthylisobutylcétone.

11. Procédé selon une ou plusieurs des revendications précédentes, caractérisé en qu'on effectue une étape f) supplémentaire comme suit :
f) on fait réagir avec un alcool le mélange d'acide méthacrylique/eau obtenu dans l'étape e) ou de l'acide méthacrylique pur lui-même, pour obtenir comme produit, selon des méthodes connues en soi, des esters recherchés (= esters d'acide méthacrylique) sous une forme désirée pour l'utilisation.

12. Procédé selon la revendication 11, **caractérisé en ce que** l'alcool est le méthanol, l'éthanol, le propanol et des composés homologues et analogues correspondants d'hydrocarbures contenant jusqu'à C₁₂.

13. Procédé selon une ou plusieurs des revendications 1 à 12 précédentes, **caractérisé en ce qu'**en un temps de réaction pour l'amidation inférieur à 60 min, et en un temps de réaction pour l'hydrolyse inférieur à 120 min, on parvient à des taux de conversion totale de plus de (>) 99 %.

14. Procédé selon une ou plusieurs des revendications 1 à 12 précédentes, **caractérisé en ce qu'**on effectue la réaction en un temps de réaction pour l'amidation inférieur à 30 min, et en un temps de réaction pour l'hydrolyse inférieur à 100 min.

15. Procédé selon une ou plusieurs des revendications 1 à 12 précédentes, **caractérisé en ce qu'**on effectue la réaction en un temps de réaction pour l'amidation inférieur à 20 min, et en un temps de réaction pour l'hydrolyse inférieur à 75 min.
